# EUROPEAN PATENT SPECIFICATION

(11) **EP 2 922 479 B1**
(45) Date of publication and mention of the grant of the patent: **18.10.2017**
(21) Application number: 13856085.9
(22) Date of filing: 21.11.2013
(51) Int. Cl.: A61B 10/02

(54) **BREAST BIOPSY LATERAL ARM SYSTEM**
BRUSTBIOPSIE-QUERLENKERSYSTEM
SYSTÈME DE BRAS LATÉRAL POUR BIOPSIE DU SEIN

(30) Priority: 21.11.2012 US 201261729141 P
(43) Date of publication of application: 30.09.2015
(73) Proprietor: Hologic Inc., Marlborough, MA 01752 (US)
(72) Inventor: DEFREITAS, Kenneth, F., Patterson, NY 12563 (US); DEYOUNG, Thomas, W., Hopewell Junction, NY 12533 (US); GIRGENTI, John, New Milford, CT 06776 (US); STANGO, Timothy, R., Sandy Hook, CT 06482 (US)
(74) Representative: Hoyng Rokh Monegier LLP
(86) International application number: PCT/US2013/071139
(87) International publication number: WO 2014/081908

(56) References cited:
- WO-A1-02/41794
- WO-A1-2013/040017
- WO-A2-00/28882
- US-A- 5 078 140
- US-A- 5 280 427
- US-A- 5 628 327
- US-B1- 6 665 554
- US-B1- 6 712 773

## Description

### BACKGROUND

The subject matter of this application is generally related to the medical field. Medical imaging technologies such as stereotactic x-ray, fluoroscopy, computer tomography, ultrasound, nuclear medicine and magnetic resonance imaging enable detection of small abnormalities in the body of a patient. The discovery of certain abnormalities may prompt performance of a biopsy procedure to obtain a tissue sample for lab analysis to help diagnose and treat patients suspected of having cancerous tumors, premalignant conditions or other diseases or disorders. A stereotactic guided percutaneous breast biopsy is often preferable to an open surgical breast biopsy in the case of small abnormalities located deep within the body because a percutaneous biopsy removes a relatively small amount of tissue. For example, a needle can be used to remove individual cells or clusters of cells in the case of fine needle aspiration (FNA), and a core or fragment of tissue in the case of a core biopsy. One difficulty associated with such procedures is that it is necessary to locate the biopsy needle with considerable precision in order to obtain a suitable sample of a small abnormality, particularly if it is located deep within the body. Moreover, it would be advantageous if the medical equipment for performing such procedures were practical to manufacture at a reasonable cost and usable by medical personnel without extensive training.

Patent application WO-A-02/41794 discloses an apparatus for assisting percutaneous light beam guided biopsy. Patent application WO-A-00/28882 discloses an apparatus that can be used for accurately locating a medical tool with respect to a patient disposed inside an imaging device. WO2013/0400147, prior art under Art. 54(3) EPC, discloses a biopsy device including a lateral member having an axis, a gun mount configured to support a biopsy gun and movable along the axis of the lateral member.

### SUMMARY

In accordance with an aspect, an apparatus comprises: a lateral arm; a carnage assembly which traverses along the lateral arm in a predefined axis, the carriage assembly including a carriage and self-adjusting rollers which are loaded against the lateral arm by spring members; and a gun mount configured to support a biopsy gun, the gun mount being connected to the carriage. The self-adjusting rollers may include a set of fixed guide rollers on a first side of the lateral arm and a movable set of guide rollers on a second side of the lateral arm. One or more spring members may be used to load the movable set of guide rollers against the lateral arm. The spring member may include a resilient beam. A cam-actuated X-axis stop may be removably secured to the lateral arm to limit an extent of traverse of the carriage.

In accordance with another aspect, an apparatus comprises: a lateral arm; a carriage which traverses along the lateral arm in a predefined axis; a cam-actuated lock assembly which secures the carriage to the lateral arm in an engaged state; and a gun mount configured to support a biopsy gun, the gun mount being connected to the carriage. Movement of the carriage relative to the lateral arm may be controlled by a lead screw associated with the lateral arm and carriage nut attached to the carriage, and the lock assembly may include a clamp which applies frictional force to the lead screw in response to actuation of a camshaft, thereby inhibiting rotation of the lead screw. A clamp bushing may be disposed between the clamp and the lead screw. A cam-actuated X-axis stop may be removably secured to the lateral arm to limit an extent of traverse of the carriage.

In accordance with another aspect, an apparatus comprises: a lateral arm; a carriage which traverses along the lateral member in a predefined axis; a gun mount configured to support a biopsy gun, the gun mount being connected to the carriage; and a cam-actuated locking mechanism for securing the gun mount to the carriage. A positional offset feature may be provided which repositions the gun mount along a secondary axis which is orthogonal to the predefined axis. The locking mechanism may include a tee nut associated with the carriage and a corresponding tee slot associated with the gun mount. A camshaft can be used to actuate the tee nut to apply frictional force against the tee slot in an engaged state. The camshaft may actuate the tee nut in the engaged state by allowing a spring to apply force directly to the tee nut, such that the camshaft does not apply force against the tee slot via the tee nut. A camshaft may actuate a locking pilot pin associated with the carriage, the pin applying force against the gun mount. The camshaft may actuate the locking pilot pin in an engaged state by allowing a spring to apply force directly to the locking pilot pin, such that the camshaft does not apply force against the gun mount via the locking pilot pin. The carriage may include at least one stop pin and the gun mount may include at least one stop pin seat, such that the gun mount is aligned in a predetermined relationship with the carriage when the stop pin is fully inserted into the stop pin seat. First and second sets of stop pin seats may be provided such that the first set of stop pin seats align the gun mount in a position offset by 180 degrees from an alignment position determined by the second set of stop pin seats.

In accordance with another aspect, a method comprises: securing a biopsy needle to a gun mount associated with a carriage; and positioning the biopsy needle for a procedure by traversing the carriage along a lateral arm in a predefined axis, including the carriage riding on self-adjusting rollers which are loaded against the lateral arm by spring members. The self-adjusting rollers may include a set of fixed guide rollers on a first side of the lateral arm and a movable set of guide rollers on a second side of the lateral arm. At least one spring member may be used to load the movable set of guide rollers against the lateral arm. The spring member may include a resilient beam. A cam-actuated X-axis stop may be secured to the lateral arm to limit an extent of traverse of the carriage.

In accordance with another aspect, a method comprises: securing a biopsy needle to a gun mount associated with a carriage; and positioning the biopsy needle for a procedure by traversing the carriage along the lateral arm in a predefined axis; and securing the carriage to the lateral arm via a cam-actuated lock assembly. Movement of the carriage relative to the lateral arm may be controlled by a lead screw associated with the lateral arm and carriage nut attached to the carriage, and the lock assembly may include a clamp which applies frictional force to the lead screw in response to actuation of a camshaft, thereby inhibiting rotation of the lead screw. A clamp bushing may be disposed between the clamp and the lead screw. A cam-actuated X-axis stop may be secured to the lateral arm to limit an extent of traverse of the carriage.

In accordance with another aspect, a method comprises: securing a biopsy needle to a gun mount via a cam-actuated locking mechanism, the gun mount associated with a carriage; and positioning the biopsy needle for a procedure by traversing the carriage along the lateral arm in a predefined axis. A positional offset feature may be provided to reposition the gun mount along a secondary axis which is orthogonal to the predefined axis. The locking mechanism may include a tee nut associated with the carriage and a corresponding tee slot associated with the gun mount. A camshaft can be used to actuate the tee nut to apply frictional force against the tee slot in an engaged state. For example, the camshaft may actuate the tee nut in the engaged state by allowing a spring to apply force directly to the tee nut, such that the camshaft does not apply force against the tee slot via the tee nut. A camshaft can be used to actuate a locking pilot pin associated with the carriage, the pilot pin applying force against the gun mount. The camshaft may actuate the locking pilot pin in an engaged state by allowing a spring to apply force directly to the locking pilot pin, such that the camshaft does not apply force against the gun mount via the locking pilot pin. The carriage may include at least one stop pin and the gun mount may include at least one stop pin seat, such that the gun mount is aligned in a predetermined relationship with the carriage when the stop pin is fully inserted into the stop pin seat. First and second sets of stop pin seats may be provided such that the first set of stop pin seats align the gun mount in a position offset by 180 degrees from an alignment position determined by the second set of stop pin seats.

Unless specifically stated otherwise, the features described herein can be used in any combination, and the aspects can include any one or more of the embodiments. Moreover, other features and advantages will become apparent to those of ordinary skill in the art in view of the figures and detailed description.

### BRIEF DESCRIPTION OF THE FIGURES

Figures 1 and 2 are isometric and side views, respectively, of one embodiment of a breast biopsy station according to the present invention, the breast biopsy station being shown in Figure 2 together with a breast;
Figure 3 is an isometric view of the integrated biopsy needle guidance system of the breast biopsy station of Figure 1;
Figure 4 is a cross-sectional view taken along line K-K of Figure 5A, illustrating the roller guide wheels of the carriage assembly and the generally V-shaped grooves of the lateral arm of the needle guide system in greater detail;
Figure 5A is a side view of certain components of the integrated biopsy needle guidance system shown in Figure 3;
Figure 5B is a bottom view of certain components of the integrated biopsy needle guidance system shown in Figure 3;
Figure 6A is a partly exploded perspective view of the combination of the carriage slide locking assembly and the carriage assembly shown in Figure 5B;
Figure 6B is a section view taken along line P-P of Figure 5A;
Figure 7A is an end view of the combination of the gun mount and the carriage assembly of Figure 3, the roller guide wheels of the carriage assembly not being shown, the locking knob of the carriage assembly being shown in an unlocked or disengaged position;
Figure 7B is a top view of the carriage assembly of Figure 7A, the roller guide wheels of the carriage assembly not being shown, the locking knob of the carriage assembly being shown in an unlocked or disengaged position;
Figure 7C is a top view of the gun mount shown in Figure 7A, together with the needle guide;
Figures 7D and 7E are cross-sectional views corresponding to Figures 7B and 7C, respectively, taken along section lines B1-B1 and B1.1-1.1;
Figure 7F is a cross-sectional view of the carriage assembly of Figure 7B taken along section line B2-B2;
Figure 8A is an end view of the combination of the gun mount and the carriage assembly of Figure 3, the roller guide wheels of the carriage assembly not being shown, the locking knob of the carriage assembly being shown in a locked or engaged position;
Figure 8B is a top view of the combination of the gun mount and the carriage assembly shown in Figure 8A;
Figure 8C is a cross-sectional view of the combination of the gun mount and the carriage assembly shown in Figure 8B taken along section line C1-C1;
Figure 8D is a cross-sectional view of the combination of the gun mount and the carriage assembly shown in Figure 8B taken along line section C2-C2;
Figure 9 is an isometric view of the X-axis stop shown in Figure 3;
Figure 10A is a top view of the X-axis stop of Figure 9 shown in the unlocked position;
Figure 10B is a cross-sectional view of the X-axis stop of Figure 10A taken along section line D1-D1;
Figure 10C is a side view of the X-axis stop of Figure 9 in the unlocked position;
Figure 10D is a top view of the X-axis stop of Figure 9 in the locked position.
Figure 10E is a cross-sectional view of the X-axis stop of Figure 10D taken along section line D2-D2;
Figure 10F is a side view of the X-axis stop of Figure 9 in the locked position;
Figure 10G is a section view showing the X-axis stop of Figure 9 mounted on the lateral arm of Figure 3; and
Figures 11 through 18 illustrate various configurations of the lateral arm needle guide and assembly steps of various components.

### DETAILED DESCRIPTION

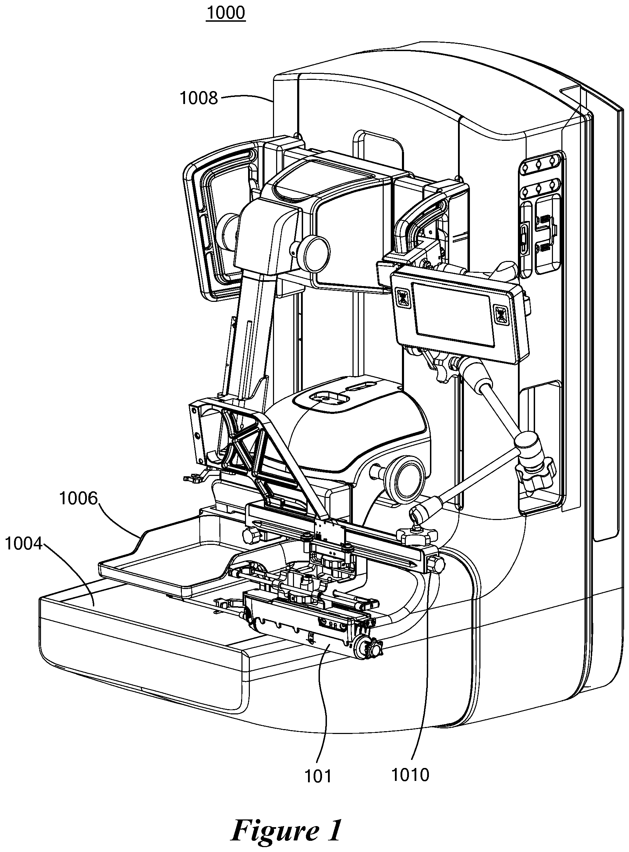

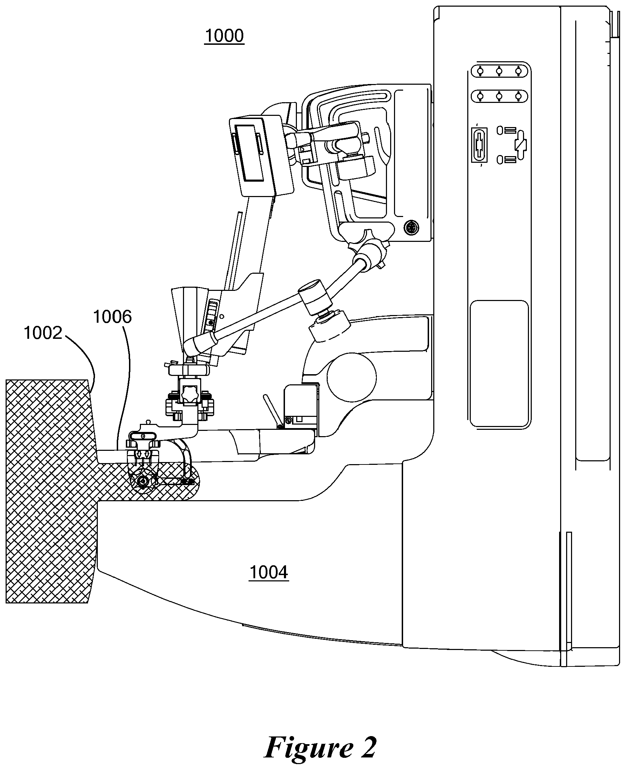

Referring to Figures 1 and 2, there is shown one embodiment of a breast biopsy station according to the present invention, the breast biopsy station being represented generally by reference numeral 1000. Breast biopsy station 1000 may comprise a breast platform 1004, a compression paddle 1006, a radiographic imaging system 1008, and an integrated biopsy needle guidance system 1010. As seen best in Figure 2, breast platform 1004 and compression paddle 1006 may be arranged so that the chest wall 1002 of a patient may be pressed against breast platform 1004 and compression paddle 1006, with the patient's breast being positioned on top of breast platform 1004 and under compression paddle 1006, i.e., the breast may be compressed between breast platform 1004 and compression paddle 1006. Radiographic imaging system 1008 may be used to produce an image of the breast that is compressed between breast platform 1004 and compression paddle 1006 in order to locate a feature of interest, e.g., a lesion, located within the breast. Integrated biopsy needle guidance system 1010 may be used to obtain a tissue sample of the feature of interest. More particularly, integrated biopsy needle guidance system 1010 may display information about the relative locations of the targeted feature and a biopsy gun 101 in order to help position biopsy gun 101 and to guide its path of travel such that a needle on biopsy gun 101 may intersect with the target feature.

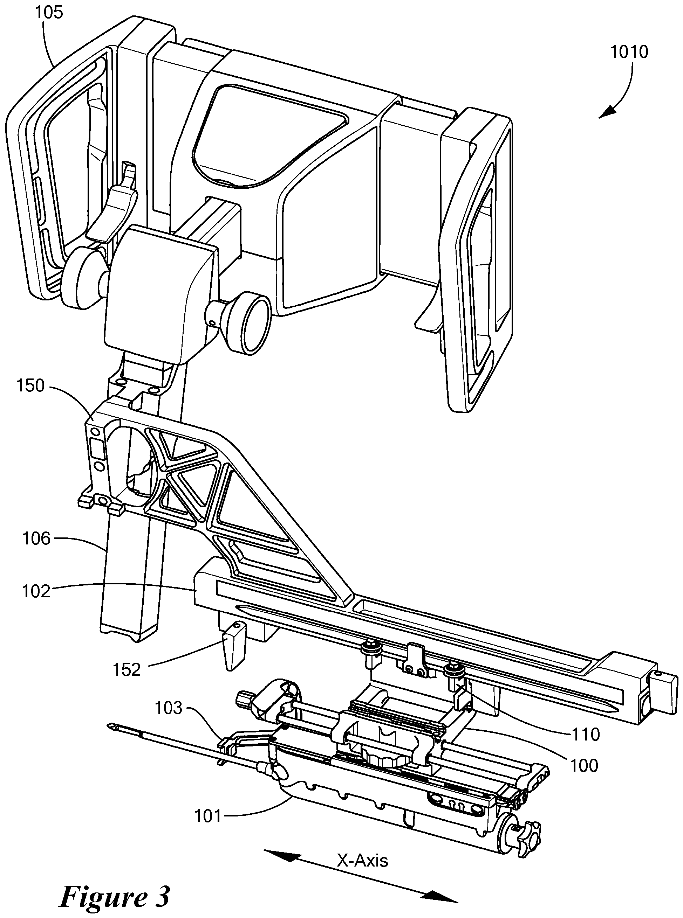

Referring now to Figure 3, there is shown integrated biopsy needle guidance system 1010 in greater detail. Integrated biopsy needle guidance system 1010 may comprise a gun mount 100, a carriage assembly 110, a lateral arm 102, and a biopsy guidance module 105. Biopsy gun 101 may be connected to gun mount 100. Gun mount 100 may be connected to carriage assembly 110. Carriage assembly 110 may be connected to lateral arm 102. Lateral arm 102 may be connected to biopsy guidance module 105. Biopsy guidance module 105 may include interface features which help calculate, utilize and display useful information. Reconfiguration features may be provided to allow secure repositioning of the selected biopsy gun, and sensing features such as described in U.S. Patent Application No. 13/611,502, published as US 9,308,017, inventors Girgenti et al., filed September 12, 2012, titled Breast Biopsy Lateral Arm System, may be provided to detect certain reconfiguration and repositioning data in order to facilitate the biopsy procedure, e.g., by providing the data to the biopsy guidance module in order to automate calculation of offsets, adjustments and other information that helps to obtain tissue cores from a specific location. Moreover, the data can be used to verify that a configuration entered by an operator matches the actual configuration of the needle guidance system.

A selected biopsy gun 101 may be positioned and secured by gun mount 100. More particularly, an operator can securely mount and remove any of various biopsy guns that might be selected, so different biopsy guns may be utilized as needed. Indexing features may help assure that the biopsy gun can be predictably and repeatedly mounted in a particular position with respect to the gun mount when mounted. Biopsy gun 101 may be operative in response to information from an embedded computer, information provided by an operator via biopsy guidance module 105, and sensor input to locate a biopsy needle of biopsy gun 101 to obtain a tissue sample, e.g., by inserting an outer cannula into a predetermined location of interest within the patient, extracting a tissue core sample by moving an inner cannula relative to the outer cannula, and removing the needle from the patient. A needle guide 103 may help to guide the outer cannula of the biopsy gun, e.g., by inhibiting deflection. Various types of biopsy guns and needles are known, and the functioning of biopsy guns and needles in obtaining tissue cores is well understood in the art. Therefore, these features will not be described in greater detail.

One aspect of positioning biopsy gun 101 relative to the patient may be via movement of lateral arm 102. Biopsy guidance module 105 may include a post member 106. Lateral arm 102 may include a clamp member 150 which can be slidably moved along and secured to post member 106 at any of various locations along the length of post member 106. As a result, biopsy gun 101 may be repositionable via movement of lateral arm 102 with respect to post member 106.

Another aspect of positioning the biopsy gun 101 relative to the patient may be via movement of biopsy gun 101 relative to lateral arm 102. Carriage assembly 110 may be selectively movable (e.g., slidably) along an X-axis defined by lateral arm 102, to which carriage assembly 110 may be connected. When biopsy gun 101 is secured to gun mount 100, and gun mount 100 is secured to carriage assembly 110, biopsy gun 101 may be positioned at any of various points along the X-axis by moving carriage assembly 110 with respect to lateral arm 102. A carriage slide locking feature may enable an operator to secure carriage assembly 110 to lateral arm 102 when a desired X-axis position is attained, thereby securing biopsy gun 101 in a desired X-axis position relative to the patient. Moreover, an X-axis stop 152 may be used to reliably return biopsy gun 101 to a particular X-axis position.

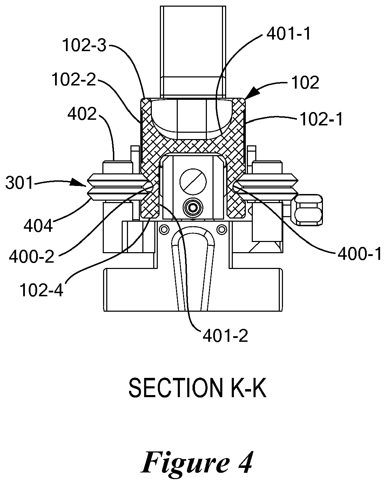

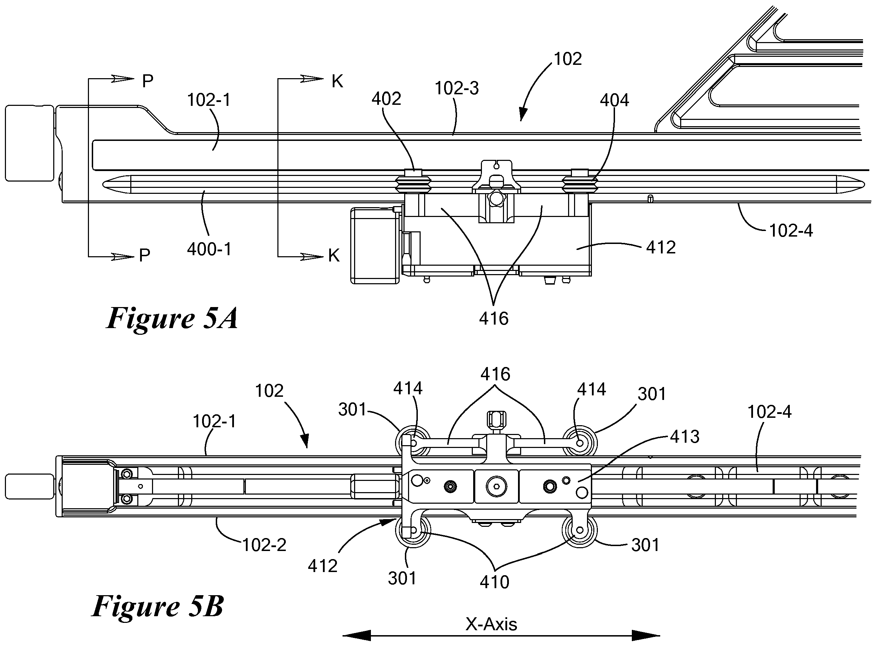

Referring now to Figures 4, 5A and 5B, lateral arm 102 may comprise an elongated member that may be generally H-shaped in transverse cross-section. Lateral arm 102 may be shaped to include a first side surface 102-1, a second side surface 102-2, a top surface 102-3, and a bottom surface 102-4. A first generally V-shaped groove 400-1 with a rounded vertex may be provided in first side surface 102-1 of lateral arm 102, and a second generally V-shaped groove 400-2 with a rounded vertex may be provided in second side surface 102-2 of lateral arm 102. First and second generally V-shaped grooves 400-1 and 400-2 may extend for at least a portion of the length of lateral arm 102, with first and second generally V-shaped grooves 400-1 and 400-2 extending parallel to one another along the X-axis of lateral arm 102. A top channel 401-1 may be provided in top surface 102-3 of lateral arm 102, and a bottom channel 401-2, the purpose of which will become apparent below, may be provided in bottom surface 102-4 of lateral arm 102.

Carriage assembly 110 may comprise a carriage 412. Carriage 412, in turn, may comprise a structure, optionally a unitary structure, shaped to include a central body 413, a set of fixed guide roller mounts 410 positioned on a first side of central body 413, and a set of self-adjusting guide roller mounts 414 positioned on a second side of central body 413. As will be discussed further below, fixed guide roller mounts 410 and self-adjusting guide roller mounts 414 may be used to mount guide rollers 301 that, in turn, may engage lateral arm 102 by seating against generally V-shaped grooves 400-1 and 400-2. More specifically, self-adjusting guide roller mounts 414 may be positioned proximate to first side surface 102-1 of lateral arm 102, with its associated guide rollers 301 seated against generally V-shaped groove 400-1, and fixed guide roller mounts 410 may be positioned proximate to second side surface 102-2 of lateral arm 102, with its associated guide rollers 301 seated against generally V-shaped groove 400-2. Self-adjusting guide roller mounts 414 may be connected to one another by deflecting beams 416. Deflecting beams 416 may allow self-adjusting guide roller mounts 414 to flex laterally relative to central body 413 whereas fixed guide roller mounts 410 may not move relative to central body 413. Deflecting beams 416 may be resilient and/or may serve to apply a spring force to help keep guide rollers 301 seated against generally V-shaped grooves 400-1 and 400-2, thereby compensating for dimensional variations associated with the machining processes of related parts. Deflecting beams 416 may be formed by machining carriage 412 in a manner that leaves two thin beams of aluminum or other suitable material which are designed to deflect to a pre-determined position and apply a pre-determined force when deflected. The deflection creates a preload force that helps keep guide rollers 301 engaged against generally V-shaped grooves 400-1 and 400-2. However, a wide variety of means of applying spring force might also be utilized.

As noted above, guide rollers 301 may allow carriage 412 to travel along the X-axis of lateral arm 102 while inhibiting movement of carriage 412 outside the X-axis. Each guide roller 301 may include a bearing 402 and a wheel 404. Wheel 404 may be appropriately shaped to contact its respective generally V-shaped groove 400-1 or 400-2 and to center guide roller 301 therewithin. Wheel 404 may be shaped to include a central groove, which may be generally V-shaped, generally U-shaped, or any other of a wide variety of shapes. The outer edges of wheel 404 may be chamfered so that contact with generally V-shaped groove 400-1 or 400-2 does not prevent wheel 404 from centering within the generally V-shaped groove. Wheel 404 may be connected to carriage 412 via bearing 402, which may enable wheel 404 to turn as carriage assembly 110 is moved along the X-axis of lateral arm 102.

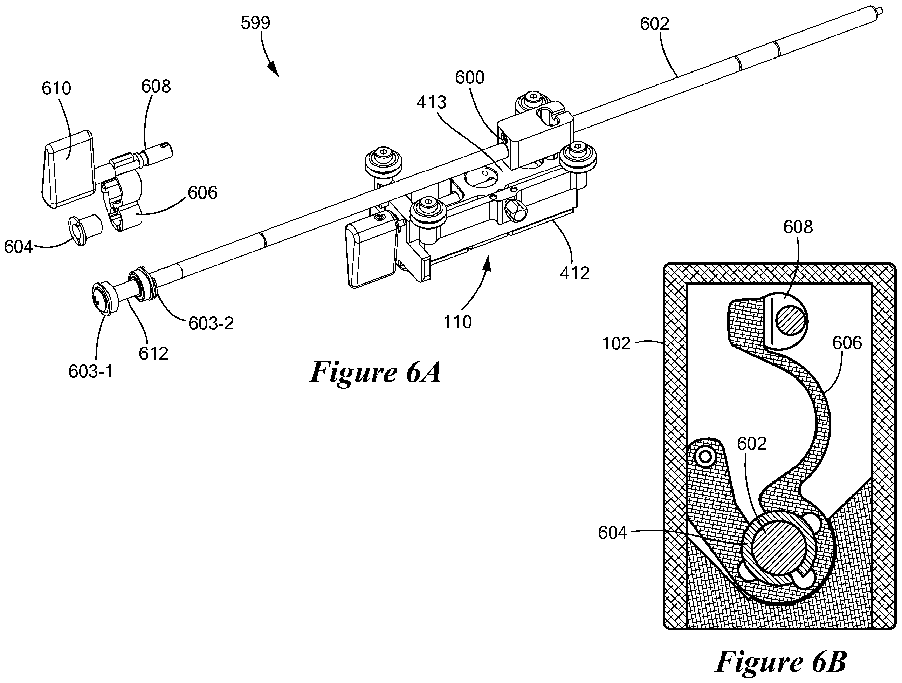

Referring now to Figure 6A, there is shown the combination of a carriage slide locking assembly 599 and carriage assembly 110. As will become apparent from the discussion below, carriage slide locking assembly 599 may be constructed so that, when in an unlocked state, carriage locking assembly 599 allows carriage assembly 110 to move freely along the x-axis of lateral arm 102, and so that, when in a locked state, carriage locking assembly 599 secures carriage assembly 110 to lateral arm 102 at a desired position along the x-axis of lateral arm 102. Carriage locking assembly 599 may comprise a carriage nut 600, a lead screw 602, lead screw bearings 603-1 and 603-2, a clamp bushing 604, a clamp 606, a camshaft 608, and a carriage locking knob 610. Carriage nut 600 may be fixed to the top of central body 413 of carriage 412 and may be appropriately dimensioned to slide longitudinally back and forth within channel 401-2 of lateral arm 102. Lead screw 602 may be coupled to lateral arm 102 via lead screw bearings 603-1 and 603-2 in such a way that lead screw 602 may rotate about its longitudinal axis while otherwise being fixed to lateral arm 102. Lead screw 602 may extend through a longitudinal bore in carriage nut 600. Carriage nut 600 and lead screw 602 may have complementary internal and external threads (not shown) so that, as lead screw 602 is rotated, carriage nut 600 is caused to move along the x-axis of lead screw 602, the direction of X-axis movement being a function of the direction of rotation of lead screw 602.

Once carriage nut 600 has been positioned at a desired location along the x-axis of lead screw 602, further rotation of lead screw 602 may be limited by the combination of clamp bushing 604, clamp 606, camshaft 608, and carriage locking knob 610. More specifically, clamp bushing 604 may be positioned coaxially around an unthreaded clamping portion 612 of lead screw 602 that is located between lead screw bearings 603-1 and 603-2. One end of clamp 606 may be positioned proximate to clamp bushing 604, and the opposite end of clamp 606 may be coupled to camshaft 608. Camshaft 608, which may be mechanically coupled to and actuated by rotation of carriage locking knob 610, may include a machined section which may be asymmetrical with respect to the axis about which camshaft 608 rotates. In the unlocked state, camshaft 608 does not apply any force to clamp 606 due to the asymmetry of the machined section. As a result, clamp 606 does not cause clamp bushing 604 to grip lead screw 602 at clamping surface 612; consequently, lead screw 602 is free to rotate, and carriage assembly 110 may move along the x-axis of lateral arm 102. By contrast, in the locked state (see Figure 6B), the asymmetrical cam section on camshaft 608 bears down on clamp 606, causing clamp 606 to compress clamp bushing 604 against lead screw 602, thereby preventing lead screw 602 from rotating due to frictional force between bushing 604 and clamping surface 612. Such a lack of rotation of lead screw 602 keeps carriage nut 600 from moving and, thereby, prevents carriage assembly 110 from moving along the x-axis of lateral arm 102.

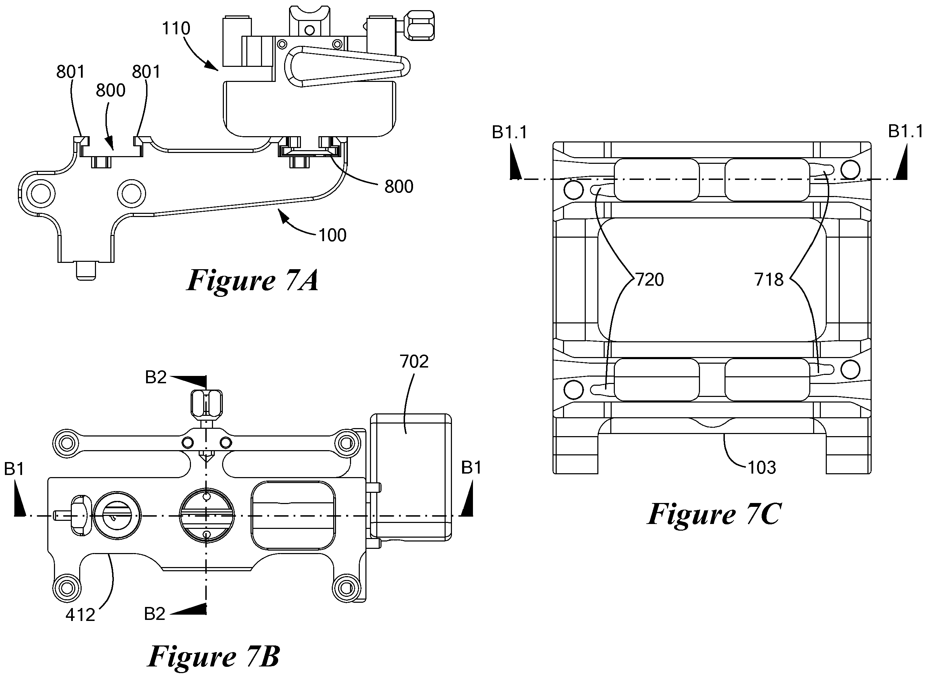

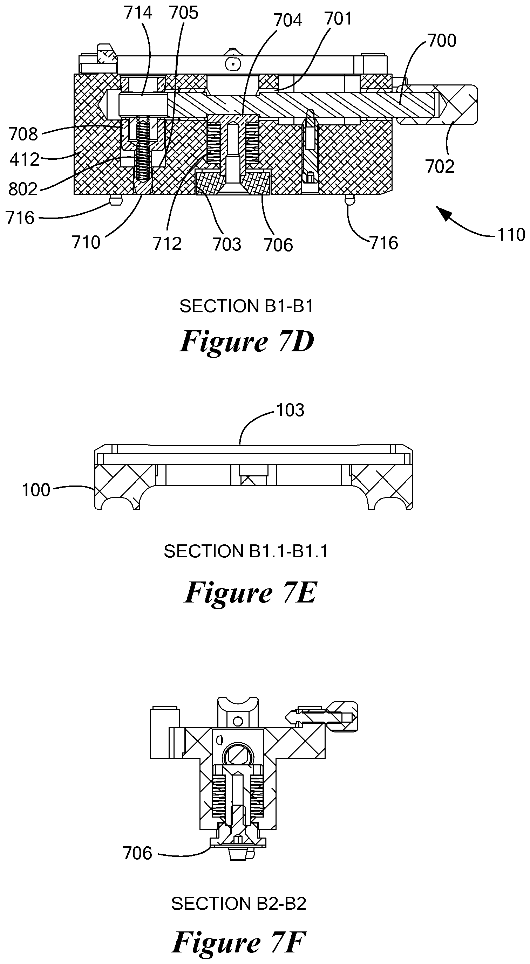

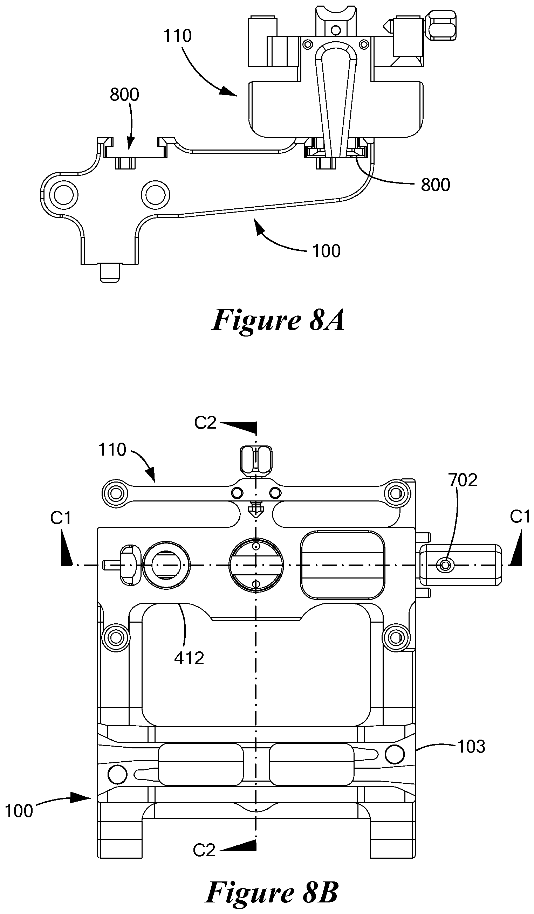

Referring now to Figures 7A through 7F and to Figures 8A through 8D, the manner in which carriage assembly 110 and gun mount 100 may be reversibly and lockably interconnected is made apparent. In particular, Figure 7A shows carriage assembly 110 and gun mount 100 interconnected in an unlocked state, and Figures 8A through 8D show carriage assembly 110 and gun mount 100 interconnected in a locked state.

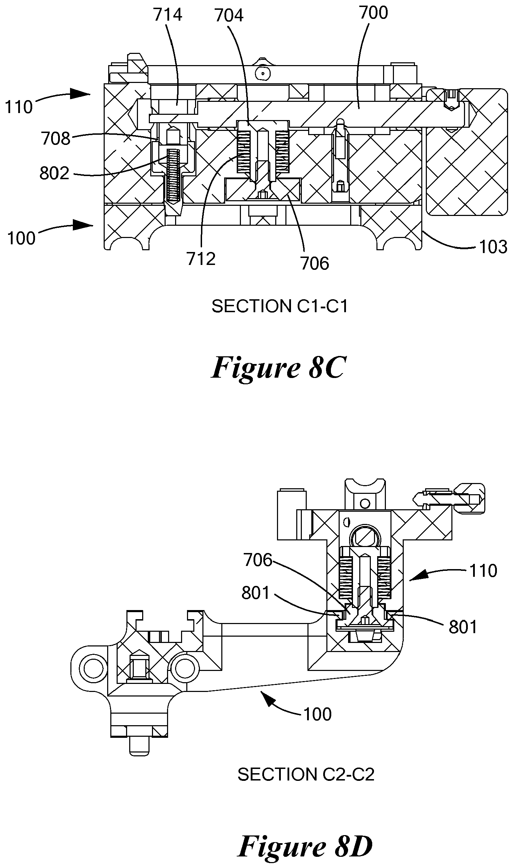

As can be seen, for example, in Figure 7D, carriage assembly 110 may further comprise a camshaft 700. Camshaft 700 may be rotatably mounted within a cavity 701 that may be provided in carriage 412. A locking knob 702, which may be disposed outside of carriage 412, may be mechanically coupled to camshaft 700 and may be used to rotate camshaft 700. Carriage assembly 110 may further comprise a tee nut 706. Tee nut 706 may be movably mounted in a vertical direction relative to a cavity 703 that may be provided in carriage 412. Vertical movement of tee nut 706 relative to cavity 703 may be controlled by camshaft 700, a tee nut cam follower 704, and tee nut springs 712. More specifically, camshaft 700 and tee nut cam follower 704 may be sized and shaped so that, when camshaft 700 is in an unlocked or disengaged position (i.e., locking knob 702 horizontal), camshaft 700 causes tee nut cam follower 704 to cause tee nut springs 712 to be compressed, thereby lowering tee nut 706 to its lowest operating position (see, for example, Figure 7F). By contrast, when camshaft 700 is in a locked or engaged position (i.e., locking knob 702 vertical), camshaft 700 causes tee nut cam follower 704 to cause tee nut springs 712 not to be compressed, thereby raising tee nut 706 to its highest operating position (see, for example, Figure 8C).

Carriage assembly 110 may further comprise a locking pilot pin 710. Locking pilot pin 710 may be movably mounted in a vertical direction into and partially out of a cavity 705 that may be provided in carriage 412. Vertical movement of locking pilot pin 710 may be controlled by a secondary cam 714 on camshaft 700, a secondary lock cam follower 708, and a secondary lock spring 802. More specifically, secondary cam 714 and secondary lock cam follower 708 may be sized and shaped so that, when camshaft 700 is an unlocked or disengaged position (i.e., locking knob 702 horizontal), locking pilot pin 710 is raised to a position that is entirely within cavity 705 (see, for example, Figure 7D). By contrast, when camshaft 700 is in a locked or engaged position (i.e., locking knob 702 vertical), locking pilot pin 710 is driven down to a position that extends partially out of cavity 705 (see, for example, Figure 8C).

Carriage assembly 110 may further comprise one or more X-axis stop pins 716 fixedly mounted on the bottom of carriage 412. X-axis stop pins 716 may be used to mate with X-axis stop pin seats 718 provided in gun mount 100 in such a way that gun mount 100 may be properly aligned with carriage 412 only when stop pins 716 are disposed in stop pin seats 718. Such positioning facilitates calculations associated with biopsy needle positioning relative to the patient. An additional set of X-axis stop pin seats 720 may be provided in gun mount 100 to allow gun mount 100 to be mounted in a 180 degree offset position.

To couple gun mount 100 to carriage assembly 110, locking knob 702 may be placed in a horizontal position (toward either side). Placement of locking knob 702 in such a horizontal position orients camshaft 700 so that tee nut cam follower 704 compresses tee nut springs 712, thereby lowering tee nut 706 to its lowest operating position. In addition, placement of locking knob 702 in such a horizontal position also causes locking pilot pin 710 to be raised into cavity 705 of carriage 412. With tee nut 706 thus lowered and locking pilot pin 710 thus raised, carriage assembly 110 may then be coupled to gun mount by sliding the bottom end of carriage 412, as well as tee nut 706, into one of the two tee slots 800 provided in gun mount 100 until stop pins 716 are seated within their corresponding stop pin seats 718. Locking knob 702 may then be turned to its vertical position (i.e., an approximately 90 degree rotation) in order to lock gun mount 100 to carriage assembly 110. The rotation of camshaft 700 in response to the rotation of locking knob 702 enables tee nut cam follower 704 to be raised by tee nut springs 712. This, in turn, causes tee nut 706 to be raised. Tee nut 706, in turn, grabs the flanges 801 of gun mount 100 and clamps flanges 801 against the bottom of carriage 412, thereby locking gun mount 100 against carriage 412. The rotation of camshaft 700 in response to moving locking knob 702 to a vertical position also allows secondary cam 714 (now driven down by secondary lock spring 802) and, thus, locking pilot pin 710 to lower down and engage gun mount 100, preventing gun mount 100 from being removed from carriage assembly 110.

To decouple gun mount and carriage assembly 110, locking knob 702 is moved from a vertical position to a horizontal position. Such movement causes locking pilot pin 710 to be raised, so that locking pilot pin 710 no longer engages gun mount 100. In addition, such movement also causes tee nut 706 to be lowered, thereby releasing the grip of tee nut 706 on gun mount 100. As a result of the foregoing, carriage assembly 110 can be slid away from gun mount 100.

Figures 7A, 7C, 8A, 8B and 8D illustrate a Y-axis offset feature. More specifically, gun mount 100 can be attached to carriage 412 via either of two tee slots 800 which are offset in the Y-axis. The two mounting positions defined by the Y-axis offset allow a biopsy needle to be positioned farther back from the chest wall of the patient. This advantageously accommodates a wider range of breast sizes, e.g., the offset position for larger breasts.

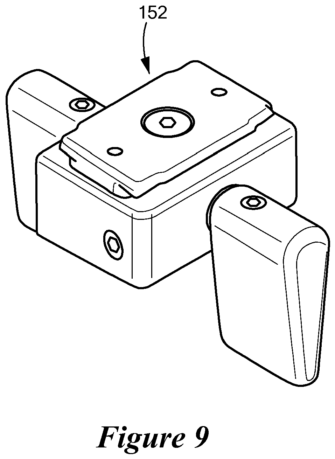

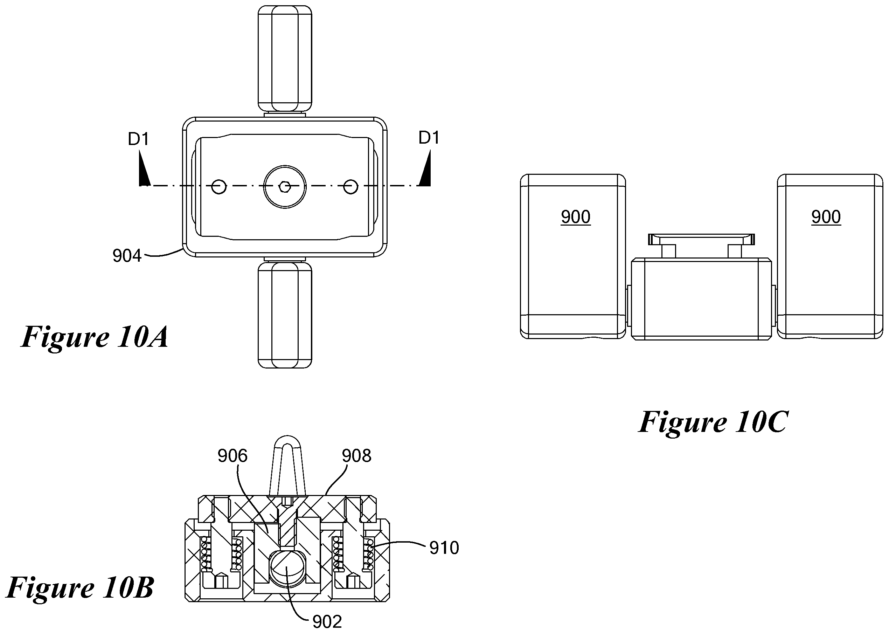

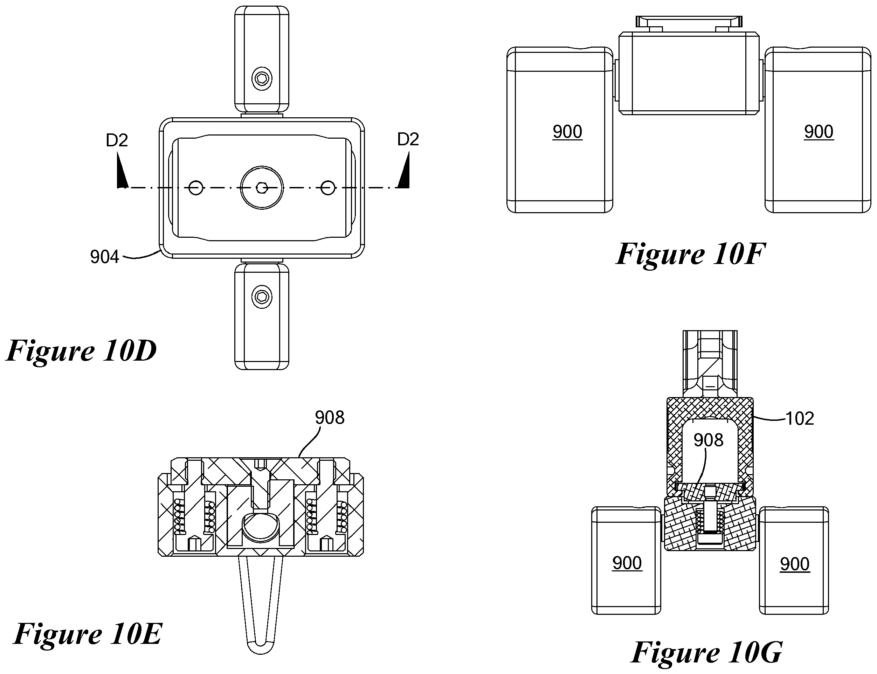

Referring now to Figures 9, 10A, 10B, 10C, 10D, 10E and 10F, X-axis stop 152 is shown in greater detail. X-axis stop 152 may be removably mounted in bottom channel 401-2 of lateral arm 102 (see Figure 10G) to provide a hard stop for delimiting x-axis movement of carriage assembly 110 relative to lateral arm 102. Corresponding locking knobs 900 are connected via a locking cam 902 which is disposed in an opening machined through the x-axis stop body 904. In the unlocked state specifically shown in Figures 10A, 10B and 10C, the locking knobs 900 are in the upper vertical position. In this position, the locking cam 902 exerts force against the locking cam follower 906, thereby lifting the tee nut 908 and compressing the locking springs 910. This is the highest operating position of the tee nut 908, which creates enough clearance between flanges of the tee nut and the X-axis stop body so that the assembly can be installed onto and move freely within a tee slot of the lateral arm 102. In the locked state specifically shown in Figures 10D, 10E and 10F, the locking knobs 900 are in the lower vertical position (rotated 180 degrees relative to the unlocked position). In this position, the locking cam 902 is no longer applying force against the locking cam follower 906 so the locking springs 910 are free to retract the tee nut 908 toward the X-axis stop body 904, thereby clamping against lateral arm 102. Note that the likelihood of premature wear due to excess clamping force is mitigated by use of the locking springs rather than direct pressure from the knobs.

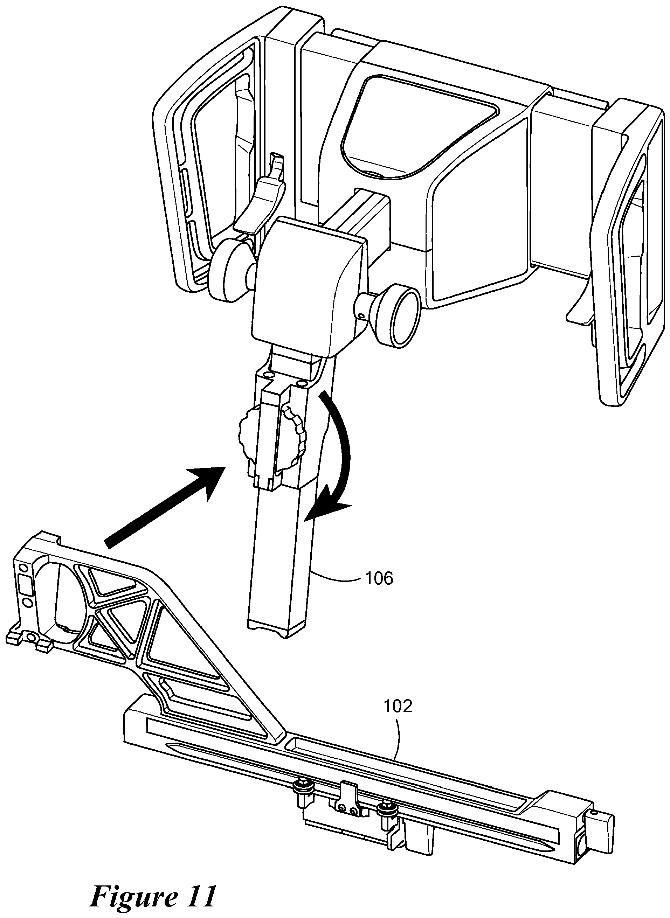

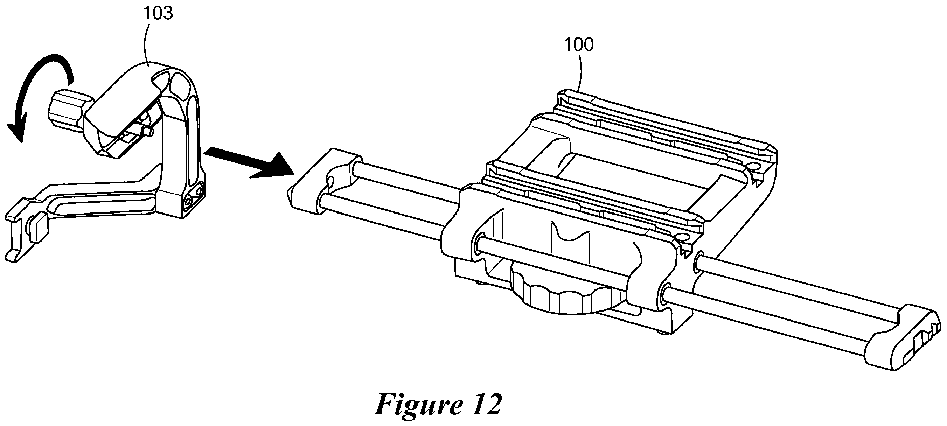

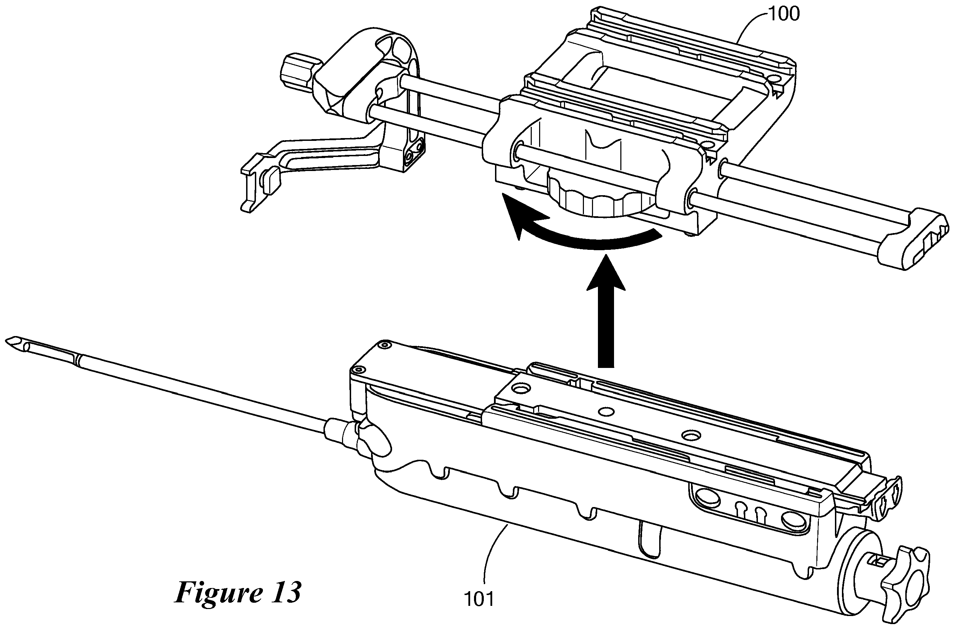

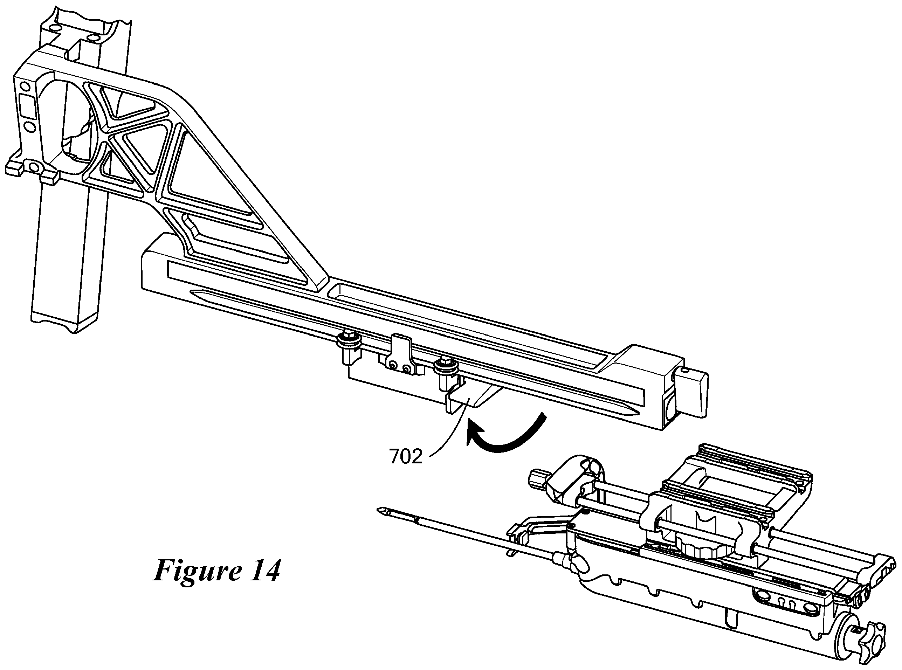

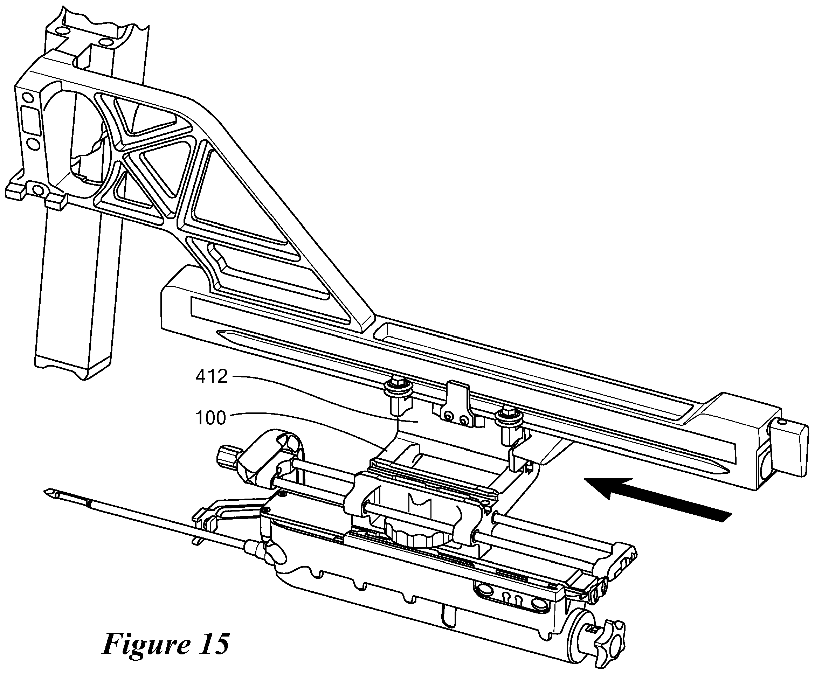

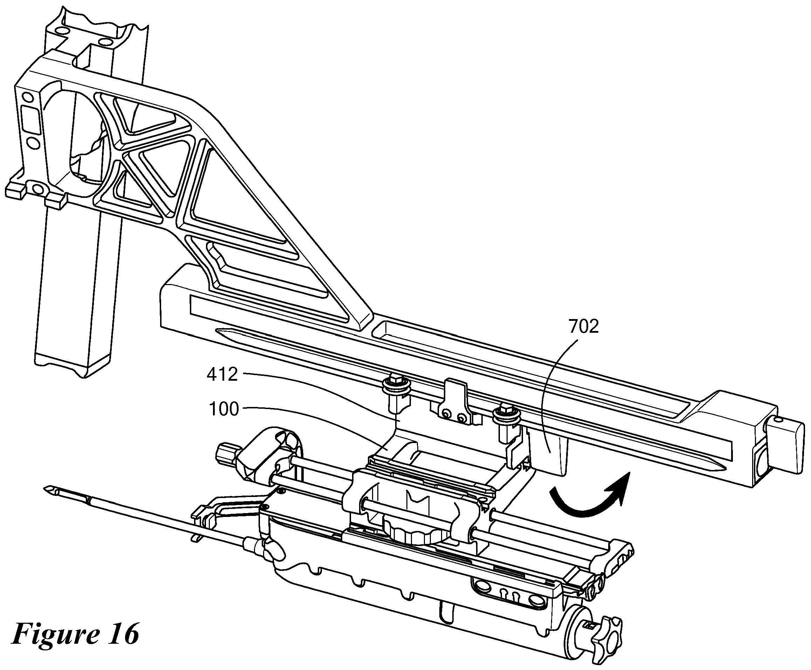

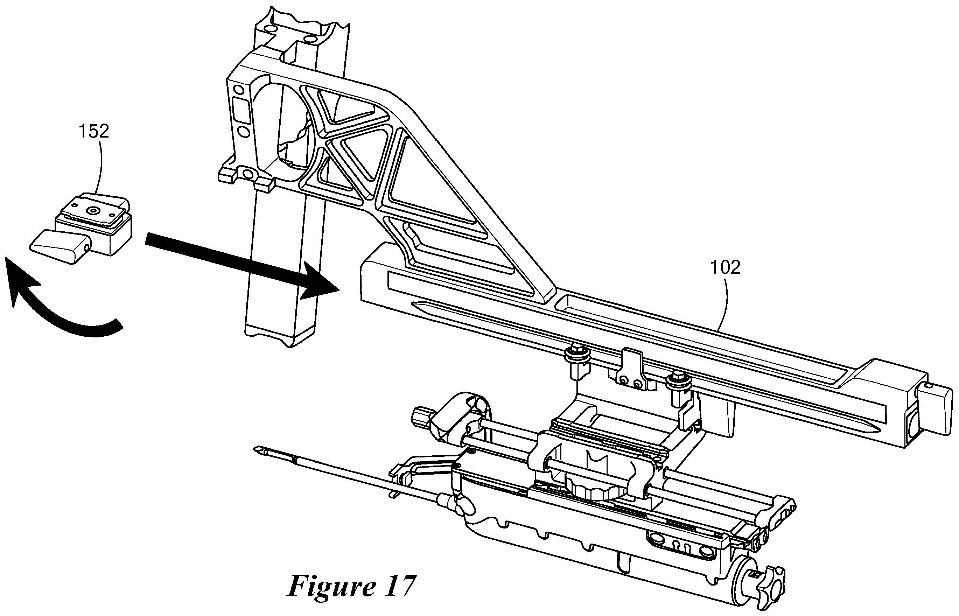

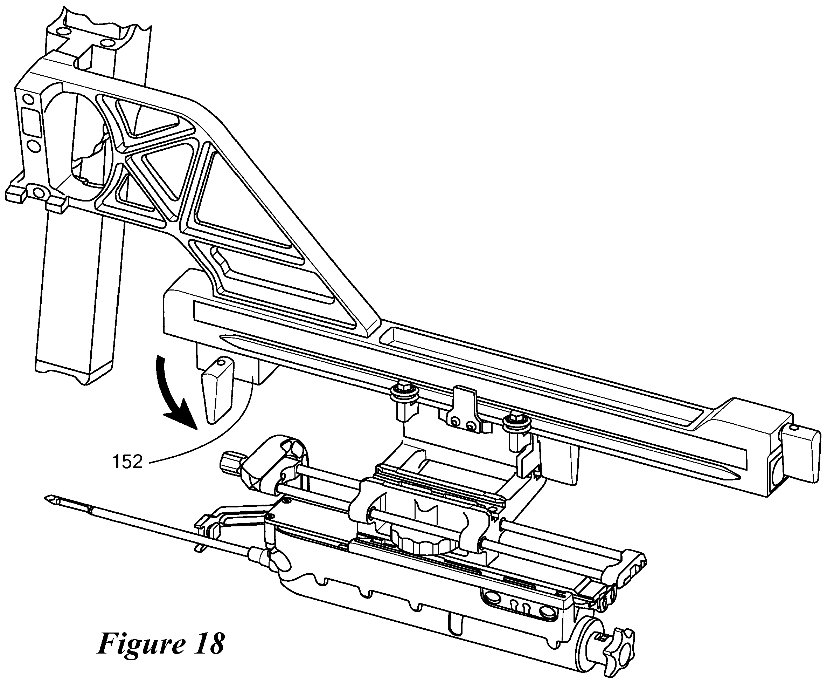

Figures 11 through 18 illustrate various configurations of the lateral arm needle guide and assembly steps of various components. Figure 11 shows lateral arm 102 being secured to post member 106. Figure 12 shows needle guide 103 being attached to gun mount 100. Figure 13 shows biopsy needle gun 101 being attached to gun mount 100. Figure 14 shows gun mount locking knob 702 being positioned in the disengaged state. Figure 15 shows gun mount 100 being slid into a mounted position with respect to carriage 412. Figure 16 shows gun mount locking knob 702 being positioned in the engaged state, thereby securing gun mount 100 to carriage 412. Figure 17 shows X-axis stop 152 being positioned in the disengaged state and mounted onto lateral arm 102. Figure 18 shows X-axis stop 152 being positioned in the engaged state, thereby securing it in position relative to the lateral arm 102.

The present invention is set out in the following claims. The embodiments, aspects or examples according to the present description that do not fall within the scope of said claims are provided for illustrative purposes only and do not form part of the present invention.

## Claims

1. A biopsy needle guidance system (1010) comprising:
- a lateral arm (102);
- a carriage assembly (110) which traverses along the lateral arm (102) in a predefined axis, the carriage assembly (110) including a carriage (412) and self-adjusting rollers (301) which are loaded against the lateral arm (102) by spring members when the carriage assembly (110) traverses along the lateral arm (102); and
- a gun mount (100) configured to support a biopsy gun, the gun mount being connected to the carriage.

2. The biopsy needle guidance system of claim 1, wherein the self-adjusting rollers (301) include a set of fixed guide rollers on a first side of the lateral arm and a movable set of guide rollers on a second side of the lateral arm, and wherein at least one spring member loads the movable set of guide rollers against the lateral arm, and preferably wherein the spring member includes a resilient beam.

3. The biopsy needle guidance system of claim 1 or 2, further including a cam-actuated X-axis stop (152), which is removably secured to the lateral arm to limit an extent of traverse of the carriage.

4. The biopsy needle guidance system of any of the previous claims comprising a cam-actuated lock assembly (599) which secures the carriage to the lateral arm in an engaged state.

5. The biopsy needle guidance system of claim 4 wherein the movement of the carriage relative to the lateral arm is controlled by a lead screw associated with the lateral arm and carriage nut attached to the carriage, and wherein the cam-actuated lock assembly includes a clamp which applies frictional force to the lead screw in response to actuation of a camshaft, thereby inhibiting rotation of the lead screw.

6. The biopsy needle guidance system of any of the previous claims comprising a cam-actuated locking mechanism (700,702,706,712,710) for securing the gun mount to the carriage.

7. The biopsy needle guidance system of claim 6, further including a positional offset feature which repositions the gun mount along a secondary axis which is orthogonal to the predefined axis.

8. The biopsy needle guidance system according to claim 6 or 7, wherein the locking mechanism includes a tee nut (706) associated with the carriage and a corresponding tee slot associated with the gun mount, preferably wherein a camshaft (700) actuates the tee nut (706) to apply frictional force against the tee slot in an engaged state.

9. The biopsy needle guidance system according to any of the claims 6-8, wherein the carriage (110) includes at least one stop pin (716) and the gun mount includes at least one stop pin seat (718,720), and wherein the gun mount is aligned in a predetermined relationship with the carriage when the stop pin is fully inserted into the stop pin seat.

10. The biopsy needle guidance system according to any of the claims 6-9, further including first and second sets of stop pin seats , the first set of stop pin seats aligning the gun mount in a position offset by 180 degrees from an alignment position determined by the second set of stop pin seats.

11. A method for positioning a biopsy needle for a procedure using a biopsy needle guidance system comprising a lateral arm (102); a gun mount (100) associated with a carriage (110) configured to traverse along the lateral arm (102) in a predefined axis and self-adjusting rollers (301) which are loaded against the lateral arm (102) by spring members; the method comprising:
- securing the biopsy needle to the gun mount (100) associated with the carriage (110); and
- positioning the biopsy needle for the procedure by traversing the carriage (110) along the lateral arm (102) in a predefined axis, the carriage (110) riding on the self-adjusting rollers (301) which are loaded against the lateral arm (102) by the spring members when the carriage (110) traverses along the lateral arm (102).

12. The method of claim 11, wherein the self-adjusting rollers include a set of fixed guide rollers on a first side of the lateral arm and a movable set of guide rollers on a second side of the lateral arm, wherein at least one spring member loads the movable set of guide rollers against the lateral arm.

13. The method of claim 10 or 11, including securing a cam-actuated X-axis stop to the lateral arm to limit an extent of traverse of the carriage.

14. The method of any of the claims 10-13 comprising securing the carriage to the lateral arm via a cam-actuated lock assembly.

## Patentansprüche

1. Führungssystem für Biopsienadeln (1010), umfassend:
- einen seitlichen Arm (102);
- eine Gestellanordnung (110), die sich entlang des seitlichen Arms (1202) auf einer vorbestimmten Achse bewegt, wobei die Gestellanordnung (110) ein Gestell (412) und selbstjustierende Rollen (301) enthält, die durch Federelemente zum seitlichen Arm (102) vorgespannt sind, wenn sich die Gestellanordnung (110) entlang des seitlichen Arms (102) bewegt; und
- eine Pistolenhalterung (100), die konfiguriert ist, eine Biopsiepistole zu tragen, wobei die Pistolenhalterung mit dem Gestell verbunden ist.

2. Führungssystem für Biopsienadeln nach Anspruch 1, wobei die selbstjustierenden Rollen (301) einen Satz an fixierten Führungsrollen auf einer ersten Seite des seitlichen Arms und einen beweglichen Satz Führungsrollen auf einer zweiten Seite des seitlichen Arms enthalten, und wobei mindestens ein Federelement den beweglichen Satz Führungsrollen zum seitlichen Arm vorspannt und wobei das Federelement vorzugsweise einen elastischen Ausleger enthält.

3. Führungssystem für Biopsienadeln gemäß Anspruch 1 oder 2, das ferner einen nockenbetätigten X-Achsen-Anschlag (152) enthält, der lösbar am seitlichen Arm befestigt ist, um ein Bewegungsausmaß des Gestells einzuschränken.

4. Führungssystem für Biopsienadeln gemäß einem der vorstehenden Ansprüche, das eine nockenbetätigte Sicherungsanordnung (599) umfasst, die das Gestell in einem ergriffenen Zustand am seitlichen Arm sichert.

5. Führungssystem für Biopsienadeln gemäß Anspruch 4, wobei die Bewegung des Gestells relativ zum seitlichen Arm durch eine mit dem seitlichen Arm assoziierte Leitspindel und eine am Gestell befestigte Gestellmutter gesteuert wird, und wobei die nockenbetätigte Sicherungsanordnung ein Klemmstück enthält, das als Reaktion auf eine Betätigung der Nockenwelle eine Reibungskraft auf die Leitspindel aufbringt, wodurch eine Drehung der Leitspindel gehemmt wird.

6. Führungssystem für Biopsienadeln gemäß einem der vorstehenden Ansprüche, das einen nockenbetätigten Sicherungsmechanismus (700, 702, 706, 712, 710) zum Sichern der Pistolenhalterung am Gestell umfasst.

7. Führungssystem für Biopsienadeln gemäß Anspruch 6, das ferner ein Positionsoffsetelement enthält, das die Pistolenhalterung entlang einer sekundären Achse neu positioniert, die auf die vordefinierte Achse orthogonal ist.

8. Führungssystem für Biopsienadeln gemäß Anspruch 6 oder 7, wobei der Sicherungsmechanismus eine mit dem Gestell assoziierte T-förmige Mutter (706) und einen entsprechenden, mit der Pistolenhalterung assoziierten T-förmigen Schlitz enthält, wobei eine Nockenwelle (700) vorzugsweise die T-förmige Mutter (706) betätigt, um in einem aktivierten Zustand eine Reibungskraft auf den T-förmigen Schlitz anzuwenden.

9. Führungssystem für Biopsienadeln gemäß einem der Ansprüche 6-8, wobei das Gestell (110) mindestens einen Anschlagstift (716) enthält und die Pistolenhalterung mindestens einen Anschlagstiftsitz (718, 720) enthält, und wobei die Pistolenhalterung in einer vorbestimmten Beziehung zum Gestell ausgerichtet ist, wenn der Anschlagstift vollständig in den Anschlagstiftsitz eingeführt ist.

10. Führungssystem für Biopsienadeln gemäß einem der Ansprüche 6-9, das ferner einen ersten und einen zweiten Satz von Anschlagstiftsitzen enthält, wobei der erste Satz von Anschlagstiftsitzen die Pistolenhalterung in einer um 180 Grad von einer Ausrichtungsposition versetzten Position ausrichtet, die durch den zweiten Satz von Anschlagstiftsitzen bestimmt ist.

11. Verfahren zum Positionieren einer Biopsienadel für eine Prozedur unter Verwendung eines Führungssystems für Biopsienadeln, das einen seitlichen Arm (102); eine mit einem Gestell (110) assoziierte Pistolenhalterung (100), die konfiguriert ist, sich entlang des seitlichen Arms (102) auf einer vorbestimmten Achse zu bewegen, und selbstjustierende Rollen (301) umfasst, die durch Federelemente zum seitlichen Arm (102) hin vorgespannt sind; das Verfahren umfassend:
- Sichern der Biopsienadel an der mit dem Gestell (110) assoziierten Pistolenhalterung (100); und
- Positionieren der Biopsienadel für die Prozedur durch Bewegen des Gestells (110) entlang des seitlichen Arms (102) auf einer vorbestimmten Achse, wobei das Gestell (110) auf den selbstjustierenden Rollen (301) fährt, die durch die Federelemente zum seitlichen Arm (102) vorgespannt sind, wenn sich das Gestell (110) entlang des seitlichen Arms (102) bewegt.

12. Verfahren gemäß Anspruch 11, wobei die selbstjustierenden Rollen einen Satz an fixierten Führungsrollen auf einer ersten Seite des seitlichen Arms und einen beweglichen Satz Führungsrollen auf einer zweiten Seite des seitlichen Arms enthalten, wobei mindestens ein Federelement den beweglichen Satz Führungsrollen zum seitlichen Arm vorspannt.

13. Verfahren gemäß Anspruch 10 oder 11, das ein Sichern eines nockenbetätigten X-Achsen-Anschlags am seitlichen Arm enthält, um ein Bewegungsausmaß des Gestells einzuschränken.

14. Verfahren gemäß einem der Ansprüche 10-13, das ein Sichern des Gestells am seitlichen Arm über eine nockenbetätigte Sicherungsanordnung enthält.

## Revendications

1. Système de guidage d'aiguille de biopsie (1010) comprenant :
- un bras latéral (102) ;
- un ensemble chariot (110) qui se déplace le long du bras latéral (102) dans un axe prédéfini, l'ensemble chariot (110) comprenant un chariot (412) et des rouleaux à ajustement automatique (301) qui sont sollicités contre le bras latéral (102) par des éléments ressorts lorsque l'ensemble chariot (110) se déplace le long du bras latéral (102) ; et
- un support de pistolet (100) configuré pour supporter un pistolet de biopsie, le support de pistolet étant relié au chariot.

2. Système de guidage d'aiguille de biopsie selon la revendication 1, dans lequel les rouleaux à ajustement automatique (301) comprennent un ensemble de rouleaux de guidage fixes sur un premier côté du bras latéral et un ensemble mobile de rouleaux de guidage sur un second côté du bras latéral, et dans lequel au moins un élément ressort sollicite l'ensemble mobile de rouleaux de guidage contre le bras latéral et, de préférence, dans lequel l'élément ressort comprenant une poutre élastique.

3. Système de guidage d'aiguille de biopsie selon la revendication 1 ou 2, comprenant en outre une butée d'axe X actionnée par came (152), qui est fixée de manière amovible au bras latéral pour limiter une étendue de déplacement du chariot.

4. Système de guidage d'aiguille de biopsie selon l'une quelconque des revendications précédentes, comprenant un ensemble verrou actionné par came (599) qui fixe le chariot au bras latéral dans un état engagé.

5. Système de guidage d'aiguille de biopsie selon la revendication 4, dans lequel le mouvement du chariot par rapport au bras latéral est commandé par une vis-mère associée au bras latéral et un écrou de chariot fixé au chariot, et dans lequel l'ensemble verrou actionné par came comprenant une pince qui applique une force de friction à la vis-mère en réponse à l'actionnement d'un arbre à cames, empêchant ainsi la rotation de la vis-mère.

6. Système de guidage d'aiguille de biopsie selon l'une quelconque des revendications précédentes, comprenant un mécanisme de verrouillage actionné par came (700, 702, 706, 712, 710) pour fixer le support de pistolet au chariot.

7. Système de guidage d'aiguille de biopsie selon la revendication 6, comprenant en outre un élément de décalage de position qui repositionne le support de pistolet le long d'un axe secondaire qui est orthogonal à l'axe prédéfini.

8. Système de guidage d'aiguille de biopsie selon la revendication 6 ou 7, dans lequel le mécanisme de verrouillage comprend un écrou à pointes à enfoncer (706) associé au chariot et une fente pour pointes à enfoncer correspondante associée au support de pistolet, de préférence dans lequel un arbre à cames (700) actionnant l'écrou à pointes à enfoncer (706) pour appliquer une force de friction contre la fente pour pointes à enfoncer dans un état engagé.

9. Système de guidage d'aiguille de biopsie selon l'une quelconque des revendications 6 à 8, dans lequel le chariot (110) comprend au moins une goupille de butée (716) et le support de pistolet comprend au moins un siège de goupille de butée (718, 720), et dans lequel le support de pistolet étant aligné dans une relation prédéterminée avec le chariot lorsque la goupille de butée est entièrement introduite dans le siège de goupille de butée.

10. Système de guidage d'aiguille de biopsie selon l'une quelconque des revendications 6 à 9, comprenant en outre des premier et second ensembles de sièges de goupille de butée, le premier ensemble de sièges de goupille de butée alignant le support de pistolet dans une position décalée de 180 degrés par rapport à une position d'alignement déterminée par le second ensemble de sièges de goupille de butée.

11. Procédé pour positionner une aiguille de biopsie pour une procédure à l'aide d'un système de guidage d'aiguille de biopsie comprenant un bras latéral (102) ; un support de pistolet (100) associé à un chariot (110) configuré pour se déplacer le long du bras latéral (102) dans un axe prédéfini, et des rouleaux à ajustement automatique (301) qui sont sollicités contre le bras latéral (102) par des éléments ressorts ; le procédé comprenant :
- fixer l'aiguille de biopsie au support de pistolet (100) associé au chariot (110) ; et
- positionner l'aiguille de biopsie pour la procédure par déplacement du chariot (110) le long du bras latéral (102) dans un axe prédéfini, le chariot (110) circulant sur les rouleaux à ajustement automatique (301) qui sont sollicités contre le bras latéral (102) par les éléments ressorts lorsque le chariot (110) se déplace le long du bras latéral (102).

12. Procédé selon la revendication 11, dans lequel les rouleaux à ajustement automatique comprennent un ensemble de rouleaux de guidage fixes sur un premier côté du bras latéral et un ensemble mobile de rouleaux de guidage sur un second côté du bras latéral, dans lequel au moins un élément ressort sollicitant l'ensemble mobile de rouleaux de guidage contre le bras latéral.

13. Procédé selon la revendication 10 ou 11, comprenant fixer une butée d'axe X actionnée par came au bras latéral pour limiter une étendue de déplacement du chariot.

14. Procédé selon l'une quelconque des revendications 10 à 13, comprenant fixer le chariot au bras latéral par l'intermédiaire d'un ensemble verrou actionné par came.
